# EUROPÄISCHE PATENTSCHRIFT

(11) **EP 0 594 999 B1**
(45) Veröffentlichungstag und Bekanntmachung des Hinweises auf die Patenterteilung: **02.12.1998**
(21) Anmeldenummer: 93114671.6
(22) Anmeldetag: 13.09.1993
(51) Int. Cl.: C07F 9/72, C07F 9/09, A61K 31/285, A61K 31/66

(54) **Phospholipidderivate, die höhere Elemente der V. Hauptgruppe enthalten**
Phospholipid derivatives containing higher elements of the fifth main group
Dérivés phospholipidiques contenant des éléments supérieurs du cinquième groupe principal

(30) Priorität: 01.10.1992 DE 4233044
(43) Veröffentlichungstag der Anmeldung: 04.05.1994
(73) Patentinhaber: ASTA Medica Aktiengesellschaft, D-01277 Dresden (DE)
(72) Erfinder: Engel, Jürgen, Prof., D-63755 Alzenau (DE); Hilgard, Peter, Dr., D-33602 Bielefeld (DE); Kutscher, Bernhard, Dr., D-63477 Maintal (DE); Nössner, Gerhard, Dr., D-63069 Offenbach (DE); Stekar, Jurij, Dr., D-63069 Offenbach (DE)
(74) Vertreter: Decker, Karl-Ludwig

(56) Entgegenhaltungen:
- EP-A- 0 108 565
- ZH. OBSHCH. KHIM. (ZOKHA4,0044460X);89; VOL.59 (7); PP.1537-47 INST. ELEMENTOORG. SOEDIN.;MOSCOW; USSR (SU) Gololobov Y G et al 'Vinyl esters of phosphorus acids. XXXI. P-vinylcholines'
- CHEMICAL ABSTRACTS, vol. 114, no. 7, 18. Februar 1991, Columbus, Ohio, US; abstract no. 062221, GOLOLOBOV Y G ET AL 'Betaines of the phosphorylvinylcholine class - a new type of acetylcholinesterase inhibitors' & DOKL. AKAD. NAUK SSSR (DANKAS,00023264);90; VOL.314 (3); PP.632-6 [CHEM.] INST. ELEMENTOORG. SOEDIN.;MOSCOW; USSR (SU)
- CHEMICAL ABSTRACTS, vol. 114, no. 5, 4. Februar 1991, Columbus, Ohio, US; abstract no. 042895, GOLOLOBOV Y G ET AL '.alpha.-Alkenyl analogs of phosphorylcholines' & HETEROAT. CHEM. (HETCE8,10427163);90; VOL.1 (2); PP.123-34 A. N. NESMEYANOV INST. ORGANOELEM. COMPD.;MOSCOW; 117813; USSR (SU)
- CHEMICAL ABSTRACTS, vol. 110, no. 19, 8. Mai 1989, Columbus, Ohio, US; abstract no. 169065, GOLOBOV Y G ET AL 'Anticholine esterase activity of unsaturated analogs of choline phosphates' & DOKL. AKAD. NAUK UKR. SSR, SER. B: GEOL., KHIM. BIOL. NAUKI (DNNADO,02018454);88; (7); PP.35-9 INST. BIOORG. KHIM.;KIEV; USSR (SU)

## Beschreibung

Langkettige Alkylphosphocholine mit antimikrobieller Wirksamkeit werden von Kanetani et al., Nippon Kayaku Kaishi, 9, 1452 (1984) beschrieben.

Die Europa-Patentanmeldung 108 565 (Anmelder: Takeda) betrifft Verbindungen der allgemeinen Formel 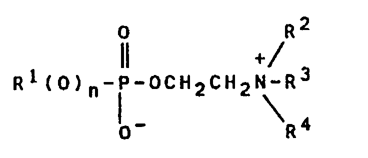
R¹ ist ein aliphatischer Kohlenwasserstoffrest mit 8 - 30 C-Atomen,
die Reste R², R² und R² sind gleich oder verschieden oder Wasserstoff oder niedere Alkylreste, oder worin die Gruppe NR²R³R⁴ eine cyclische Ammoniumgruppe bedeutet und n die Werte 0 oder 1 hat. Für diese Verbindungen wird eine Anti-Tumor-Wirkung sowie eine pilzhemmende Wirkung angegeben.

Die erfindungsgemäßen Verbindungen können zur Therapie von Tumoren und zur Behandlung von Haut- und Autoimmunkrankheiten eingesetzt werden, insbesondere die Verbindungen gemäß
Formel I, 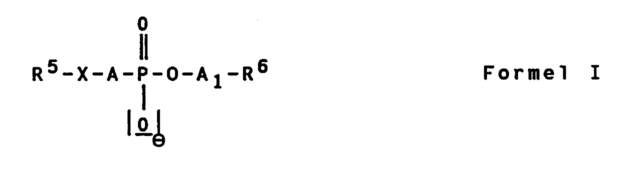 wobei
- R⁵: einen geradkettigeen oder verzweigten Alkylrest mit 10 - 24 Kohlenstoffatomen repräsentiert, der auch eine bis drei Doppel - oder Dreifachbindungen enthalten kann,
- A: stellt eine Einfachbindung oder eine der Gruppen mit den Formeln II - VI dar 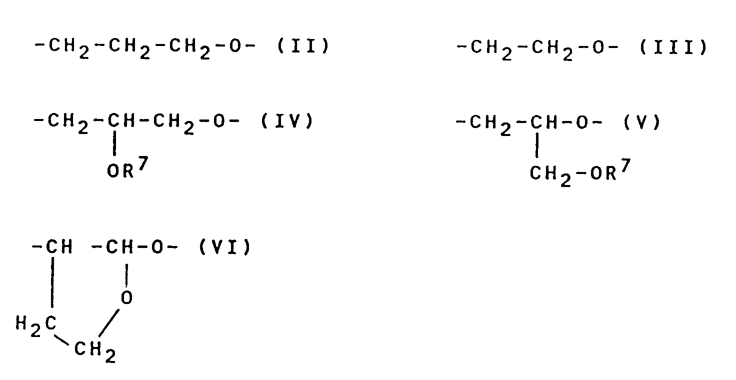
wobei
R⁷ eine geradkettige Alkylgruppe mit 1 bis 4 Kohlenstoffen, die auch verzweigt sein kann, darstellt.
   Die Gruppen (II) bis (VI) sind so orientiert, daß das Sauerstoffatom an das Phosphoratom der Verbindung (I) gebunden ist.
   - X =: Sauerstoff- oder Schwefelatom oder NH, wenn A = Einfachbindung
   - X =: Sauerstoff- oder Schwefelatom, wenn A eine Verbindung aus den Gruppen der Formeln (II) bis (VI)
   - A₁ =: geradkettiger oder verzweigter Alkylrest mit 2 bis 10 Kohlenstoffatomen, der auch ungesättigt sein kann und durch Halogen oder Hydroxygruppen substituiert sein kann.
   - R⁶=: ⁽⁺⁾YR⁸R⁹R¹⁰ mit R⁸-R¹⁰= geradkettige, verzweigte oder cyclische Alkylreste mit 1 bis 6 Kohlenstoffatomen, die gleich oder verschieden sein können, oder Wasserstoff
   - Y =: P, As, Sb oder Bi,
   oder eine Gruppe der Formel VII 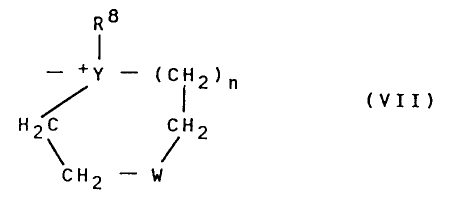
   - mit: n = 0 oder 1
   W = CH₂, O, NH oder S; unter der Voraussetzung
   n = 1, wenn W nicht gleich CH₂ ist.
   wobei Y die Bedeutungen P, As und Sb hat.

Zur Therapie von Protozoenerkrankungen eignen sich beispielsweise die Verbindungen gemäß Formel I mit Y = As und Sb. Alle Verbindungen eignen sich darüberhinaus besonders als Mittel gegen Tumorerkrankungen.

Die erfindungsgemäßen Verbindungen eignen sich zur Therapie von Bluterkrankungen, wie beispielsweise von Anämien, wie sie in Verbindung mit Tumorerkrankungen auftreten können. Desweiteren können die erfindungsgemäßen Verbindungen zur Therapie von Erkrankungen des Knochensystems, zum Beispiel Osteoporose eingesetzt werden.

Weiter können die Verbindungen gemäß Formel I zur Therapie von Viruserkrankungen und bakteriellen Infektionen eingesetzt werden.

Die Verbindungen zeichnen sich durch eine niedrige Toxizität aus.

So besitzt die Verbindung gemäß Beispiel 3 eine LD₅₀ bei einmaliger Gabe per os an der Maus von mehr als 1470 mg/kg Körpergewicht.

### Herstellung:

### Verfahren 1

Die erste Stufe des Eintopf-Verfahrens besteht in der Reaktion von Phosphoroxychlorid mit Alkohol, Thioalkohol oder Amin in halogenierten Kohlenwasserstoffen, gesättigten cyclischen Ethern, acyclischen Ethern, gesättigten Kohlenwasserstoffen mit 5 bis 10 C-Atomen, flüssigen aromatischen Kohlenwasserstoffen, die auch durch Halogen (insbesondere Chlor) substituiert sein können oder in Gemischen aus den vorstehend erwähnten Lösungsmitteln oder ohne Lösungsmittel, gegebenenfalls in Gegenwart einer hierfür üblichen basichen Substanz.
Als halogenierte Kohlenwasserstoffe kommen beispielsweise Kohlenwasserstoffe mit 1 bis 6 C-Atomen infrage, wobei ein oder mehrere oder alle Wasserstoffatome durch Chloratome ersetzt sind. Beispielsweise können Methylenchlorid, Chloroform, Ethylenchlorid, Chlorbenzol, Dichlorbenzol verwendet werden. Falls es sich um halogensubsituierte aromatische Kohlenwasserstoffe handelt, sind diese vorzugsweise mit einem oder zwei Halogenatomen substituiert.

Als gesättigte cyclische Ether können beispielsweise Ether mit einer Ringröße von 5-6, die aus Kohlenstoffatomen und einem oder 2 Sauerstoffatomen bestehen, verwendet werden.
Beispiele hierfür sind Tetrahydrofuran, Dioxan.

Die acyclischen Ether bestehen aus 2 bis 8 Kohlenstoffatomen und sind flüssig. Beispeilsweise kommen infrage: Diethylether, Diisobutylether, Methyl-tert.-butylether, Diisopropylether.

Als gesättigte Kohlenwasserstoffe kommen unverzweigte und verzweigte Kohlenwasserstoffe, die aus 5 bis 10 Kohlenstoffatomen bestehen und flüssig sind, infrage. Beispielsweise kommen Pentan, Hexan, Heptan, Cyclohexan, infrage.

Als aromatische Kohlenwasserstoffe kommen beispielsweise Benzol und alkylsubstituierte Benzole, wobei die Alkylsubstituenten aus 1 bis 5 Kohlenstoffatomen bestehen, infrage.

Als basische Substanzen sowohl für die Reaktion des Phosphoroxychlorids mit dem Alkohol, Thioalkohol oder Amin als auch für die anschließende Umsetzung mit dem Phosphonium, -Stibonium- oder Bismutoniumsalz kommen Amine infrage, beispielsweise aliphatische Amine der Formel NR⁶R⁷R⁸, wobei R⁶, R⁷ und R⁸ gleich oder verschieden sind und Wasserstoff oder C₁-C₆-Alkyl bedeuten oder auch aromatische Amine wie Pyridin oder Picolin. Weiter können beispielsweise Chinolin, Diisopropylamin, Isochinolin, Triethylamin, Hünigs Base (Hünig, Kissel, Chem. Ber., 91 (380) 1958) verwendet werden.

Bei der Umsetzung mit dem Phosphonium-, Stibonium- oder Bismutoniumsalz kann die hier erforderliche basische Substanz gleichzeitig oder auch vor dem Phosphonium-, Stibonium- oder Bismutoniumsalz zugesetzt werden. Für diese Reaktion ist in jedem Fall ein Lösungsmittel erforderlich; das heißt falls der erste Reaktionsschritt ohne ein besonderes Lösungsmittel erfolgt, muß ein solches jetzt zugegeben werden.
Das Molverhältnis von Phosphoroxychlorid zu dem Alkohol, Thioalkohol oder Amin liegt zum Beispiel zwischen 1,5:1 bis 0,8 : 1.

Falls die Reaktion des Phosphoroxychlorids mit dem Alkanol bzw. Thioalkanol oder dem Amin in Gegenwart einer basischen Substanz erfolgt, ist die Menge der basischen Substanz beispielsweise 1 bis 3 Mol bezogen auf 1 Mol POCl₃.
Für die anschließende Umsetzung mit dem Phosphonium-, Stibonium- oder Bismutoniumsalz ist die verwendete Menge an basischer Substanz beispielsweise 1 bis 5 Mol bezogen auf 1 Mol Alkohol, Thioalkohol oder Amin.

Die Reaktionstemperatur der Umsetzung von Phosphoroxychlorid mit Alkohol, Thioalkohol oder Amin mit dem Amin liegt zwischen -30^{o}C und +30^{o}C, vorzugsweise -15^{o}C und +0^{o}C, insbesondere -10^{o}C und -0^{o}C.

Die Reaktionszeit dieser Umsetzung beträgt beispielsweise 0,5-5 Stunden, vorzugsweise 1-3 Stunden, insbesondere 1,5-2 Stunden. Falls die Reaktion in Gegenwart einer basischen Substanz erfolgt, verläuft sie im allgemeinen schnell (etwa 30 Minuten).

Das erhaltene Produkt wird ohne Isolierung und Reinigung in einem inerten Lösungsmittel mit einem Phosphonium-, Arsonium-, Stibonium- oder Bismutoniumsalz der Formel VIII

OH-A₁-R⁶ Formel VIII

umgesetzt.

A₁ und R⁶ besitzen die oben angegebenen Bedeutungen.

Arsenocholin und seine Salze können beispielsweise gemäß der bei K. Irgolic, Applied Organometallic Chemistry (1987) 1, S. 403-412 angegebenen Vorschriften dargestellt werden.

Danach wird das Phosphonium-, Stibonium- oder Bismutoniumsalz portionsweise oder vollständig zugegeben.
Als Säuren des Phosphonium-, Stibonium- oder Bismutoniumsalzes kommen Salze mit Mineralsäuren, (wie zum Beispiel Schwefelsäure, Salzsäure) infrage, ferner Salze mit organischen Säuren, wie z. B. Essigsäure, para-Toluolsulfonsäure und ähnliche.

Dieser Reaktionsschritt erfolgt in einem inerten Lösungsmittel. Als Lösungsmittel kommen hier die gleichen infrage, die für die Umsetzung des Phosphoroxychlorids mit Alkohol, Thioalkohol oder Amin verwendet werden, falls diese Umsetzung in einem Lösungsmittel erfolgt.

Anschließend wird die basische Substanz in einem der angegebenen Lösungsmittel gelöst, oder ohne Lösungsmittel zugetropft.

Vorzugsweise werden als Lösungsmittel für die basische Substanz hier verwendet: Halogenierte Kohlenwasserstoffe, gesättigte cyclische Ether, acyclische Ether, gesättigte Kohlenwasserstoffe mit 5 bis 10 Kohlenstoffatomen, flüssige aromatische Kohlenwasserstoffe oder Gemische aus vorstehend erwähnten Lösungsmitteln.
Es handelt sich hier um die gleichen Lösungsmittel, wie sie für die Umsetzung des Phosphoroxychlorids mit dem Alkohol, Thioalkohol oder Amin verwendet werden können.

Durch die Zugabe der basischen Substanz steigt die Temperatur. Man sorgt dafür, daß die Temperatur in einem Bereich zwischen 0^{o}C bis 40^{o}C, vorzugsweise 10^{o}C bis 30^{o}C, insbesondere 15^{o}C bis 20^{o}C gehalten wird.

Die Reaktionsmischung wird dann noch bei 5^{o}C bis 30^{o}C, vorzugsweise 15^{o}C bis 25^{o}C gerührt, (beispielsweise 1 Stunde bis 40 Stunden, vorzugsweise 3 Stunden bis 15 Stunden.)

Die Hydrolyse des Reaktionsansatzes erfolgt durch Zugabe von Wasser, wobei eine Temperatur zwischen 10^{o}C und 30^{o}C, vorzugsweise 15^{o}C und 30^{o}C, insbesondere zwischen 15^{o}C und 20^{o}C eingehalten werden soll.

Die zuvor erwähnten Hydrolyseflüssigkeiten können auch basische Substanzen enthalten. Als solche basischen Substanzen kommen Carbonate und Hydrogencarbonate der Alkali - und Erdalkalimetalle infrage.

Zur Vervollständigung der Hydrolyse wird anschließend noch 0,5 Stunden bis 4 Stunden, vorzugsweise 1 bis 3 Stunden, insbesondere 1,5 bis 2,5 Stunden bei 10^{o}C bis 30^{o}C, vorzugsweise bei 15^{o}C bis 25^{o}C, insbesondere bei 18^{o}C bis 22^{o}C gerührt.

Die Reaktionslösung wird dann mit einem Gemisch aus Wasser und Alkoholen (vorzugsweise aliphatische gesättigte Alkohole mit 1 bis 4 Kohlenstoffatomen), das gegebenenfalls noch eine basische Substanz erhalten kann, gewaschen.
Das Mischungsverhältnis Wasser:Alkohol kann beispielsweise zwischen 5 und 0,5 liegen, vorzugsweise 1-3 (V/V).

Als basische Substanzen für die Waschflüssigkeit kommen zum Beispiel Carbonate und Hydrogencarbonate der Alkali- und Erdalkalimetalle sowie Ammoniak (zum Beispiel wässeriges Ammoniak) infrage. Besonders bevorzugt ist eine 3%ige Natriumcarbonatlösung in Wasser.

Gegebenenfalls kann anschließend eine Waschung der Reaktionslösung mit einer sauren Lösung vorgenommen werden.
Vorteilhaft ist die saure Waschung zur Entfernung noch nicht umgesetzter basischer Anteile der Reaktionslösung, insbesondere bei der Verwendung von Methylenchlorid als Lösungsmittel.

Die Waschlösung besteht aus einem Gemisch aus Wasser und Alkoholen. Vorzugsweise kommen Mischungen aus aliphatischen gesättigten Alkoholen mit 1 bis 4 Kohlenstoffatomen infrage, wobei gegebenenfalls noch eine saure Substanz anwesend sein kann. Das Mischungsverhältnis Wasser:Alkohol kann beispielsweise zwischen 5 und 0,5 liegen, vorzugsweise 1-3 (V/V).

Als saure Substanz für die Waschflüssigkeit kommen zum Beispiel Mineralsäuren und organische Säuren infrage, zum Beispiel Salzsäure, Schwefelsäure oder Weinsäure, und Zitronensäure. Besonders bevorzugt ist eine 10% Lösung von Salzsäure in Wasser.

Anschließend wird noch einmal mit einem Gemisch aus Wasser und Alkoholen gewaschen. Vorzugsweise kommen Mischungen aus aliphatischen gesättigten Alkoholen mit 1 bis 4 Kohlenstoffatomen infrage, wobei gegebenenfalls noch eine basische Substanz anwesend sein kann.

Das Mischungsverhältnis Wasser: Alkohol kann beispielsweise zwischen 5 und 0,5 liegen, vorzugsweise 1-3.

Die gewaschenen Phasen werden dann vereinigt und in üblicher Weise getrocknet, und dann das Lösungsmittel (vorzugsweise unter vermindertem Druck, zum Beispiel 5 bis 100 hPascal) gegebenenfalls nach Zugabe von 150 - 1000 ml, vorzugsweise 300 - 700 ml, insbesondere 450 - 550 ml eines aliphatischen Alkohols entfernt (bezogen auf 1 Mol Gewichtsteil trockenes Produkt).

Als Alkohole kommen vorzugsweise gesättigte aliphatische Alkohole mit einer Kettenlänge von 1 und 5 Kohlenstoffatomen in Frage. Besonders bevorzugt als Alkohole sind hierbei n-Butanol, Isopropanol. Diese Alkoholbehandlung hat den Zweck der vollständigen Entfernung von Restwasser.

Das so erhaltene Produkt kann in der üblichen Weise (z.B. durch Chromatographie, Umkristallisieren) gereinigt werden.

### Verfahren 2

besteht in der Umsetzung eines cyclischen Phosphorsäuretriesters mit einer Verbindung der Formel X oder XI.
Der cyclische Phosphorsäureester wird gemäß EP 108 565 durch Umsetzung des cyclischen Phosphorsäurediesterchlorids mit dem Alkanol erhalten.

Eine Verbindung der Formel IX 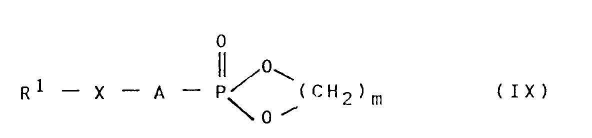 wobei m = 2 oder 3 sein kann,
wird mit einer Verbindung der Formel (X) oder (XI)

YR⁶R⁷R⁸ (X)

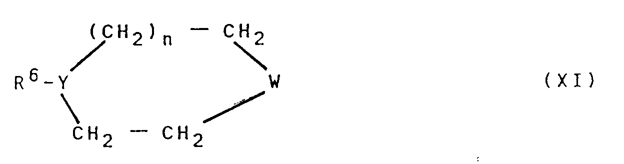

(weitere Bedeutungen siehe Formel I) in einem inerten Lösungsmittel bei erhöhter Temperatur umgesetzt.

Als inerte Lösungsmittel kommen aliphatische Nitrile in Betracht, zum Beispiel Acetonitril, Propionitril, ferner polare Lösungsmittel wie N-Methylpyrrolidon, Dimethylformamid, Dimethylacetamid.

Die Reaktionstemperatur beträgt zwischen 30°C und 140°C, vorzugsweise liegt die Reaktionstemperatur zwischen 50°C und 120°C, ganz besonders zwischen 70°C und 100°C.

Es kann sowohl bei Athmosphärendruck als auch bei erhöhtem Druck gearbeitet werden, der Druck liegt zwischen 1000 hPa und 2000 hPa, vorzugsweise zwischen 1000 hPa und 1750 hPa und ganz besonders bevorzugt zwischen 1000 hPa und 1500 hPa.

Die Reaktionsdauer liegt zwischen 0,5 h und 4 h; wenn unter erhöhtem Druck gearbeitet wird, liegt beispielsweise bei 1500 hPa und 85°C die Dauer bei 2 h.

### Verfahren 3

### Umsetzung aktivierter Derivate der Phosphorsäureester

Dieses Verfahren besteht in der Umsetzung von Hydrogenphosphat 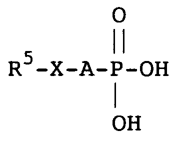 mit Verbindungen der allgemeinen Formel XIII

HO-A₁-R⁶-Z XIII

Wobei R⁵ X, A, A₁ und R⁶ die vorstehend erwähnten Bedeutungen haben
Z = stellt eine Gruppe gemäß den Formeln X oder XII dar.

HO in der Formel für das Hydrogenphosphat kann durch Halogenid, Tosylat, Mesylat und Triflat ersetzt werden.

Als wasserentziehende Mittel in der Kondensationsreaktion können Carbodiimide wie zum Beispiel Dicyclohexylcarbodiimid verwendet werden.

Als Lösungsmittel für die Verfahrensvariante kommen aprotische, polare Lösungsmittel, wie beispielsweise Acetonitril, Dimethylformamid, Dimethylacetamid, Dimethylsulfoxid, N-Methylpyrrolidon und chlorierte Kohlenwasserstoffe in Frage.

Die Temperatur beträgt beispielsweise 20 - 80^{o}, besonders bevorzugt sind 40 - 60^{o}C. Die Reaktionsdauer beträgt beispielsweise 4 Stunden.

### Verfahren 4

Eine Verbindung der allgemeinen Formel XII, die einen am aliphatischen Rest aktivierten Phosphorsäureester darstellt, 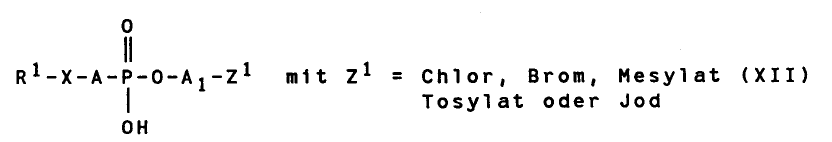 (weitere Bedeutungen wie vorstehend)
wird mit einer Verbindung der Formeln (X) oder (XI) umgesetzt.

Diese Reaktion erfolgt in bekannter Weise ohne Lösungsmittel oder in einem inerten Lösungsmittel bei Temperaturen zwischen 50^{o}C bis 150^{o}C.
Als Lösungsmittel kommen die gleichen in Frage wie für das Verfahren 2.

Im Anschluß an die Reaktion erfolgt die Umsetzung mit säurebindenden bzw. halogenid-bindenden Stoffen wie zum Beispiel Ag₂CO₃ und mit Basen, beispielsweise mit Alkalimetallcarbonaten und Erdalkalimetallcarbonaten und organischen Aminen. Als Lösungsmittel dienen aliphatische Alkohole, beispielsweise Methanol, Ethanol und Isopropanol. Eventuell kann bei erhöhter Temperatur gearbeitet werden.

Vor Abschluß aller Reaktionen ist der in der Metallorganik übliche Ausschluß von Feuchtigkeit und Luftsauerstoff einzuhalten.

### Reinigung:

Das Reinigungsverfahren kann sich an alle 4 Verfahren anschließen. Die Reinigung der oben dargestellten Verbindung kann dadurch erfolgen, daß man den Rückstand, der nach dem Verdampfen des Reaktionsmediums, gegebenenfalls unter vermindertem Druck, anfällt, in einem organischen Lösungsmittel auflöst (vorzugsweise niedere Alkohole mit einem Wassergehalt von 0-4 %, wie zum Beispiel Methanol, Ethanol, Isopropanol, n-Butanol) und mit Mischbettionenaustauscher oder nacheinander mit saurem und basischem Ionenaustauscher behandelt.
Das erhaltene Filtrat wird dann mit einem Mischbett-Ionenaustauscher, zum Beispiel Amberlite® MB3 beispielsweise 1 bis 5 Stunden, vorzugsweise 2 Stunden bei 10^{o}C bis 50^{o}C, vorzugsweise 20^{o}C gerührt. Anstelle eines Mischbett-Ionenaustauschers kann die Reinigung auch nacheinander mit einem sauren Ionenaustauscher und einem basischen Ionenaustauscher erfolgen.

Als Ionenaustauscher können alle unlösliche Feststoffe, die ionenaustauschende Gruppen enthalten, verwendet werden.

Saure Ionenaustauscher sind solche, die zum Beispiel saure Gruppen, wie Sulfonsäuregruppen, Carboxylgruppen enthalten. Beispiele sind Ionenaustauscher mit Sulfonsäuregruppen an einer Polystyrolmatrix, wie zum Beispiel Amberlite® IR 120, Dowex® HCR, Duolite® C 20 oder Lewatit® S 100. Schwach saure Ionenaustauscher sind zum Beispiel solche, die auf Basis einer Polyarcrylsäure-Matrix, wie zum Beispiel Amberlite® IRC 76, Duolite® C 433 oder Relite® CC Carbonsäuregruppen tragen.

Als basische Ionenaustauscher kommen beispielsweise solche in Frage, die an einer Polymer-Matrix (zum Beispiel Polystyrol-Matrix) primäre, sekundäre, tertiäre oder quartäre Aminogruppen tragen, wie zum Beispiel Duolite® A 101, Duolite® A 102, Duolite® A 348, Duolite® A 365, Duolite® A 375, Amberlite® IRA 67, Duolite® A 375, Amberlite® IRA 458 und Duolite® A 132.

Mischbettionenaustauscher sind Gemische aus sauren und alkalischen Ionenaustauscherharzen, wie zum Beispiel Amberlite MB1, Amberlite® MB2, Amberlite® MB3 und Amberlite® MB6.

Im übrigen wird auf Ullmann's Encyclopedia of Industrial Chemistry, 5. Auflage (1989), Band A14, S. 450 verwiesen, in dieser Stelle sind alle handelsüblichen Ionenaustauscher beschrieben, die in dem Reinigungsverfahren eingesetzt werden können.

Nach dem Absaugen vom Ionenaustauscherharz wird unter vermindertem Druck (beispielsweise 20 Torr bis 200 Torr) bei 40^{o}C bis 70^{o}C eingedampft und anschließend aus halogenierten Kohlenwasserstoffen, gesättigten aliphatischen Ketonen, Alkohol/Keton-Gemischen oder aus gesättigten oder aromatischen Kohlenwasserstoffen umkristallisiert.

Als halogenierte Kohlenwasserstoffe für die Umkristallisation kommen beispielsweise Kohlenwasserstoffe aus 1 bis 6 C-Atomen infrage, wobei ein oder mehrere oder alle Wasserstoffatome durch Chloratome ersetzt sind.
Beispielsweise können Methylenchlorid, Chloroform, Ethylenchlorid, Chlorbenzol verwendet werden.

Als Alkohole kommen gesättigte aliphatische Alkohole mit 1 bis 6 Kohlenstoffatomen und 1 bis 3 Hydroxylgruppen in Betracht. Als Ketone kommen gesättigte, aliphatische Ketone mit 3 bis 6 Kohlenstoffatomen in Betracht.

Das Mischungsverhältnis Alkohol:Keton beträgt 1 zu 1- 5 (Volumen/Volumen).
Besonders bevorzugt ist ein Ethanol/Aceton-Gemisch im Verhältnis 1 : 1 (V/V).
Als gesättigte oder aromatische Kohlenwasserstoffe kommen in Betracht: hochsiedender Petrolether, Toluol, Xylol, Ethylbenzol.

Die erfindungsgemäßen Verbindungen gemäß Formel (I) zeichnen sich durch niedrige Toxizität bei guter Anti-Tumor-Wirkung aus.
Beispielsweise verfügt die Verbindung gemäß Beispiel 1 im L1210-Zellkulturversuch über eine EC₉₀ von 2,9 µg/ml.

Die EC₉₀ ist die Konzentration einer antitumorwirksamen Substanz in µ/ml, die in vitro das Wachstum der Krebszellen um 90 % hemmt im Vergleich zu einem Kontrollversuch ohne Zugabe der antitumorwirksamen Substanz.

### Beispiel 1:

### IUPAC-Name

### 2-[[(Octadecyloxy)hydroxyphosphenyl]oxy]-As,As,As-trimethylarsonium inneres Salz

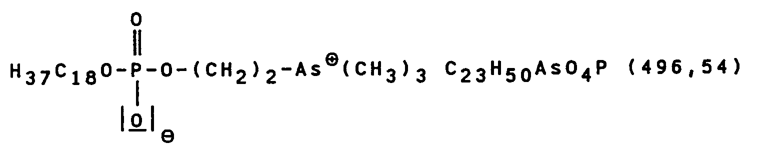

2,3 ml (25 mmol) Phosphoroxychlorid werden in 15 ml Chloroform gelöst und bei 0^{o}C - 5^{o}C tropfenweise mit einer Lösung von 6,1 g (22,5 mmol) Octadecanol in 25 ml Chloroform, das 8 ml Pyrdin enthält, versetzt. Dauer der Zugabe: 30 min bis 1 h. Man rührt noch eine Stunde bei Raumtemperatur nach und gibt anschließend in einer Portion 7,4 g (30 mmol) Arsenocholinbromid zu. In diese Lösung tropft man 10 ml Pyridin, so daß die Temperatur 20^{o}C - 25^{o}C nicht übersteigt. Nach beendeter Zugabe wird 3 h bei Raumtemperatur nachgerührt, nach dem Abkühlen auf 5^{o}C bis 10^{o}C mit 4 ml Wasser hydrolisiert und mit je 20 ml Wasser/Methanol (1:1), 3 proz. Natriumcarbonat/Methanol (1:1), 3 proz. Citronensäure/Methanol (1:1) und abschließend Wasser/Methanol (1:1) gewaschen. Die organische Phase wird über Magnesiumsulfat getrocknet, eingeengt und der Rückstand in 96 proz. Ethanol aufgenommen. Nach dem Filtrieren wird mit 30 g Ionenaustauscher Amberlite MB 3 gerührt. Man saugt über Kieselgur/Aktivkohle ab, engt im Vakuum ein und läßt unter Aceton kristallisieren.
Ausbeute: 2,54 g (23 %)

| Elementaranalyse: | | | |
|---|---|---|---|
| | C | H | As |
| ber. | 55,64 % | 10,15 % | 15,09 % |
| gef. | 55,71 % | 10,41 % | 14,1 % |
| | 55,93 % | 10,58 % | |

Dünnschichtchromatogramm:
(Chloroform/Methanol/1 M Natriumacetat in 25 proz. Ammoniak 70:40:10) RF = 0,53

### Beispiel 2

### IUPAC-Name

### 2-[[(Hexadecyloxy)hydroxyphosphenyl]oxy]-As,As,As-tri -methylarsonium inneres Salz

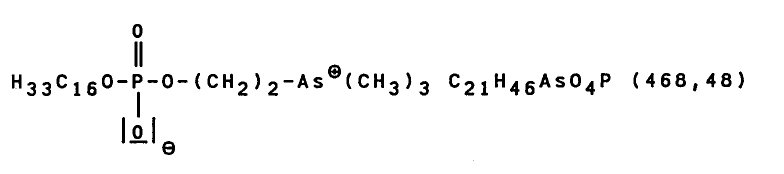

Darstellung analog Beispiel 1 aus 2,3 ml (25 mmol) Phosphoroxychlorid, 5,5 g (22,5 mmol) Hexadecanol, 8 + 10 ml Pyridin und 7,4 g (30 mmol) Arsenocholinbromid. Reinigung durch Behandeln mit 23 g Ionenaustauscher Amberlite MB3 in 96 proz. Ethanol.
Ausbeute: 1,4 g (13 %)

| Elementaranalyse: | | |
|---|---|---|
| | C | H |
| ber. | 53,84 % | 9,90 % |
| gef. | 53,45 % | 10,16 % |
| | 53,67 % | 10,20 % |

Dünnschichtchromatogramm:
(Chloroform/Methanol/1 M Natriumacetat in 25 proz. Ammoniak 70:40:10) RF = 0,48

### Beispiel 3

### IUPAC-Name

### 2-[[(cis-13-Docosenyloxy)hydroxyphosphenyl]oxy]-As,As, As-trimethylarsonium inneres Salz

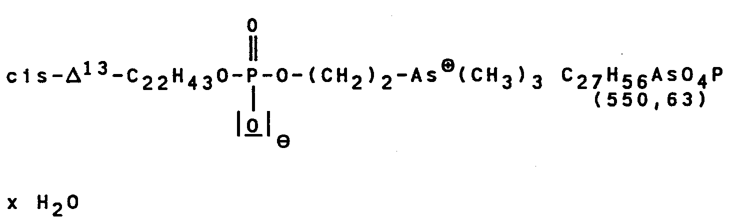

Darstellung analog Beispiel 1 aus 2,3 ml (25 mmol) Phosphoroxychlorid, 7,3 g (22,5 mmol) Erucylalkohol, 8 + 10 ml Pyridin und 7,4 g (30 mmol) Arsenocholinbromid. Reinigung durch Behandeln mit 23 g Ionenaustauscher Amberlite MB3 in 96 proz. Ethanol.
Ausbeute: 1,1 g (10 %)

| Elementaranalyse: | | |
|---|---|---|
| | C | H |
| ber. | 55,28 | 10,31 % |
| gef. | 54,98 % | 10,19 % |
| | 55,08 % | 10,30 % |

Dünnschichtchromatogramm:
(Chloroform/Methanol/1 M Natriumacetat in 25 proz. Ammoniak 70:40:10) RF = 0,52

### Beispiel 4

### IUPAC-Name

### 2-[[(Octadecyloxy)hydroxyphosphenyl]oxy]-P,P,P-trimethylphosphonium inneres Salz

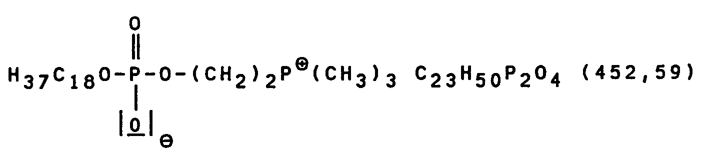

Darstellung analog Beispiel 1 aus 4,6 ml (50 mmol) Phosphoroxychlorid, 12,2 g (45 mmol) Octadecanol, 16 + 20 ml Pyridin und 12,1 g (60 mmol) Phosphocholinbromid. Reinigung durch Behandeln mit 50 g Ionenaustauscher Amberlite MB3 in 96 prozentigem Ethanol.
Ausbeute: 2,1 g (10 %)

| Elementaranalyse: | | |
|---|---|---|
| | C | H |
| ber. | 60,04 % | 11,14 % |
| gef. | 60,94 % | 11,53 % |
| | 60,44 % | 11,42 % |

Dünnschichtchromatogramm:
(Chloroform/Methanol/1 M Natriumacetat in 25 proz. Ammoniak 70:40:10) RF = 0,43

### Beispiel 5

### 2-[[(Hexadecyloxy)hydroxyphosphenyl]oxy] P,P,P-trimethylphosphonium inneres Salz

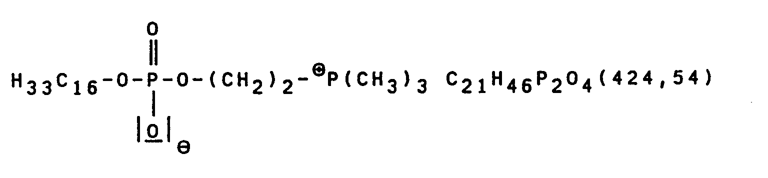

Darstellung analog Beispiel 1 aus 4,6 ml (50 mmol) Phosphoroxychlorid, 10,9 g (45 mmol) Hexadecanol, 16+20 ml Pyrdin und 12,1 g (60 mmol) Phosphocholinbromid. Reinigung durch Behandeln mit Ionenaustauscher Amberlite MB3 in 96 proz. Ethanol.
Ausbeute: 5,2 g (27 %)

| Elementaranalyse: | | |
|---|---|---|
| | C | H |
| ber. | 59,41 % | 10,92 % |
| gef. | 58,73 % | 11,16 % |
| | 59,19 % | 12,02 % |

RF = 0,47
Dünnschichtchromatogramm:
(Chloroform/Methanol/1 M Natriumacetat in 25 proz. Ammoniak 70:40:10) RF = 0,43

### Beispiel 6

### 2-[[(cis-13-Docosenyloxy)hydroxyphosphenyl]oxy] P,P,P-trimethylphosphonium inneres Salz

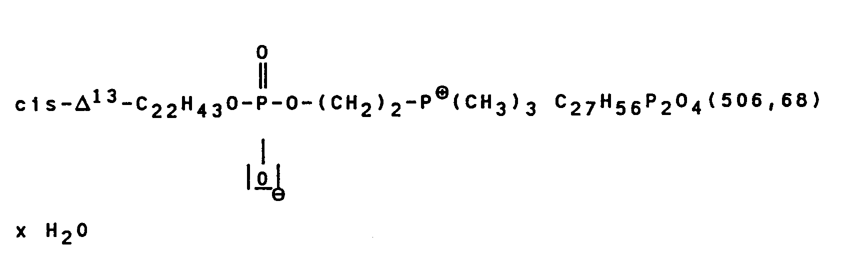

Darstellung analog Beispiel 1 aus 3,5 ml (38 mmol) Phosphoroxychlorid, 11,2 g (34 mmol) Erucylalkohol, 12 + 15 ml Pyridin und 9,2 g (46 mmol) Phosphocholinbromid. Reinigung durch Behandeln mit 45 g Ionenaustauscher Amberlite MB3 in 96 proz. Ethanol.
Ausbeute: 1,9 g (11 %)

| Elementaranalyse: | | |
|---|---|---|
| | C | H |
| ber. | 60,76 % | 11,14 % |
| gef. | 60,56 % | 11,35 % |
| | 61,39 % | 11,54 % |

Dünnschichtchromatogramm:
(Chloroform/Methanol/1 M Natriumacetat in 25 proz. Ammoniak 70:40:10) RF = 0,47

### Beispiel 7

### 2-[[(Octadecyloxy)hydroxyphosphenyl]oxy]-As,As,As-trimethylethylarsonium inneres Salz

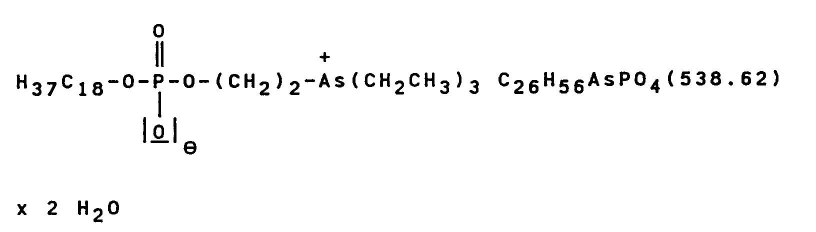

Darstellung analog Beispiel 1 aus 3,5 ml (38 mmol) Phosphoroxychlorid 9,33 g (35 mmol) Octadecanol, 12 + 15 ml Pyridin und 13,2 g (46 mmol) Triethyl-(2-hydroxyethyl)-arsoniumbromid. Reinigung durch Behandeln mit Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 3,39 g (18 %)

| Elementaranalyse: | | |
|---|---|---|
| | C | H |
| ber. | 54,35 % | 10,52 % |
| gef. | 54,65 % | 11,58 % |
| | 54,41 % | 11,78 % |

R_{F} = 0,60
Dünnschichtchromatogramm:
(Chloroform/Methanol/25 proz. Ammoniak 80:25:5)

### Beispiel 8

### 2-[[(Hexadecyloxy)hydroxyphosphenyl]oxy]-As,As,As-triethylethylarsonium inneres Salz

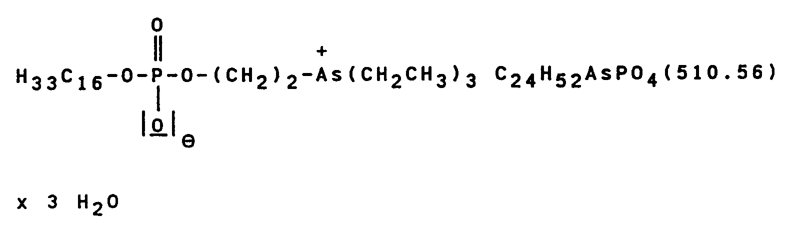

Darstellung analog Beispiel 1 aus 3,5 ml (38 mmol) Phosphoroxychlorid 8,43 g (35 mmol) Hexadecanol, 12 + 15 ml Pyridin und 13,2 g (46 mmol) Trimethyl-(2-hydroxyethyl)-arsoniumbromid. Reinigung durch Behandeln mit Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 2,26 g (13 %)

| Elementaranalyse: | | |
|---|---|---|
| | C | H |
| ber. | 51,05 % | 10,35 % |
| gef. | 50,22 % | 11,37 % |
| | 50,81 % | 11,77 % |

R_{F} = 0,57
Dünnschichtchromatogramm:
(Chloroform/Methanol/25 proz. Ammoniak 80:25:5)

### Beispiel 9

### 2-[[(Cis-13-Docosenyloxy)hydroxyphosphenyl]oxy]-As,As, As-triethylethylarsonium inneres Salz

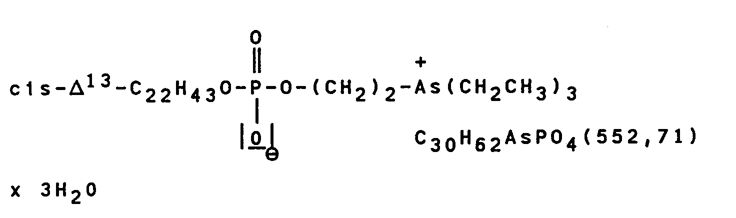

Darstellung analog Beispiel 1 aus 3,5 ml (38 mmol) Phosphoroxychlorid 11,4 g (35 mmol) Erucylalkohol, 12 + 15 ml Pyridin und 13,2 g (46 mmol) Trimethyl-(2-hydroxyethyl)-arsoniumbromid. Reinigung durch Behandeln mit 30 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 4,50 g (22 %)

| Elementaranalyse: | | |
|---|---|---|
| | C | H |
| ber. | 51,05 % | 10,35 % |
| gef. | 50,22 % | 11,37 % |
| | 50,81 % | 11,77 % |

R_{F} = 0,57
Dünnschichtchromatogramm:
(Chloroform/Methanol/25 proz. Ammoniak 80:25:5)

### Beispiel 10

### 3-[[(Octadecyl)hydroxyphosphenyl]oxy]-As,As,As-trimethylpropylarsonium inneres Salz

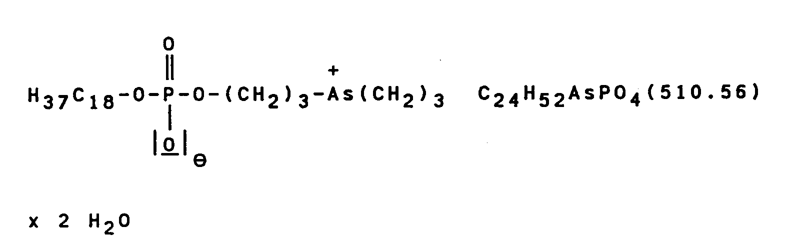

Darstellung analog Beispiel 1 aus 4,0 ml (38 mmol) Phosphoroxychlorid 10,8 g (35 mmol) Octadecanol. 14 + 17 ml Pyridin und 13,2 g (53 mmol) Trimethyl-(2-hydroxyethyl)-arsoniumbromid. Reinigung durch Behandeln mit 30 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 1,97 g (10 %)

| Elementaranalyse: | | |
|---|---|---|
| | C | H |
| ber. | 52,74 % | 10,33 % |
| gef. | 52,22 % | 10,38 % |
| | 52,19 % | 10,37 % |

R_{F} = 0,47
Dünnschichtchromatogramm:
(Chloroform/Methanol/25 proz. Ammoniak 70:40:10)

### Beispiel 11

### 3-[[(Hexadecyloxy)hydroxyphosphenyl]oxy]-As,As,As-trimethylpropylarsonium inneres Salz

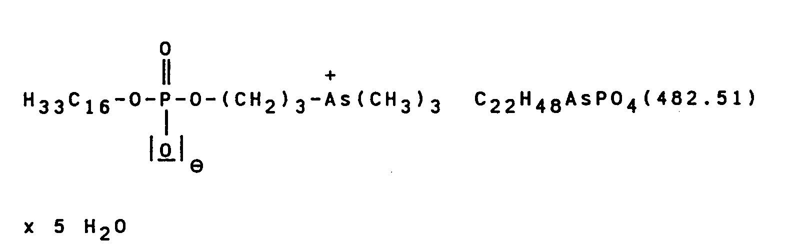

Darstellung analog Beispiel 1 aus 4,0 ml (43 mmol) Phosphoroxychlorid 9,7 g (40 mmol) Hexadecanol, 14 + 17 ml Pyridin und 13,6 g (53 mmol) Trimethyl-(3-hydroxyethyl)-arsoniumbromid. Reinigung durch Behandeln mit 30 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 2,0 g (10 %)

| Elementaranalyse: | | |
|---|---|---|
| | C | H |
| ber. | 51,86 % | 10,09 % |
| gef. | 51,55 % | 10,05 % |
| | 51,74 % | 10,17 % |

R_{F} = 0,47
Dünnschichtchromatogramm:
(Chloroform/Methanol/25 proz. Ammoniak 70:40:10)

### Beispiel 12

### 3-[[(cis-13-Docosenyloxy)hydroxyphosphenyl]oxy]-As,As, As-trimethylpropylarsonium inneres Salz

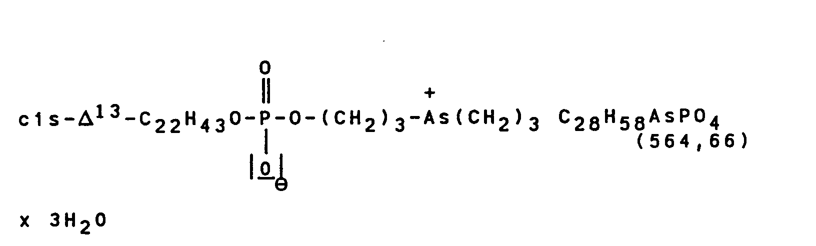

Darstellung analog Beispiel 1 aus 4,0 ml (43 mmol) Phosphoroxychlorid 13,0 g (40 mmol) Erucylalkohol 14 + 17 ml Pyridin und 13,2 g (53 mmol) Trimethyl-(3-hydroxyethyl)-arsoniumbromid. Reinigung durch Behandeln mit 30 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 2,4 g (11 %)

| Elementaranalyse: | | |
|---|---|---|
| | C | H |
| ber. | 54,36 % | 10,43% |
| gef. | 54,45 % | 10,34 % |
| | 54,86 % | 10,51 % |

R_{F} = 0,50
Dünnschichtchromatogramm:
(Chloroform/Methanol/25 proz. Ammoniak 70:40:10)

### Beispiel 13

### 3-[[(cis-13-Docosenyloxy)hydroxyphosphenyl]oxy] P,P,P-trimethylpropylphosphonium inneres Salz

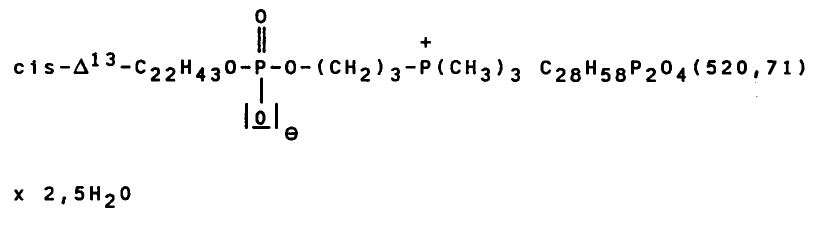

Darstellung analog Beispiel 1 aus 4,2 ml (45 mmol) Phosphoroxychlorid 13,8 g (42 mmol) Erucylalkohol 14 + 18 ml Pyridin und 12,0 g (56 mmol) Trimethyl-(3-hydroxypropyl-arsoniumbromid. Reinigung durch Behandeln mit 45 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 2,93 g (13 %)

| Elementaranalyse: | | |
|---|---|---|
| | C | H |
| ber. | 59,44 % | 11,22 % |
| gef. | 59,28 % | 10,92 % |
| | 59,57 % | 11,54 % |

R_{F} = 0,25
Dünnschichtchromatogramm:
(Chloroform/Methanol/(1 M Natriumacetat in 25 proz. Ammoniak 70:40:10)

### Beispiel 14

### 3-[[(Octadecyloxy)hydroxyphosphenyl]oxy]-P,P,P-trimethylpropylphosphonium inneres Salz

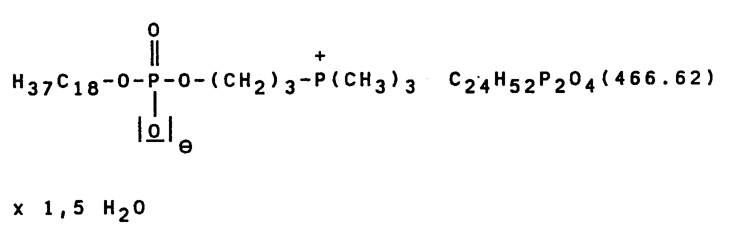

Darstellung analog Beispiel 1 aus 4,2 ml (45 mmol) Phosphoroxychlorid 11,4 g (42 mmol) Octadecanol, 14 + 18 ml Pyridin und 12,2 g (56 mmol) Trimethyl-(3-hydroxypropyl-phosphoniumbromid. Reinigung durch Behandeln mit 55 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 2,27 g (11 %)

| Elementaranalyse: | | |
|---|---|---|
| | C | H |
| ber. | 58,39 % | 11,23 % |
| gef. | 59,08 % | 11,42 % |
| | 58,63 % | 11,39 % |

R_{F} = 0,50
Dünnschichtchromatogramm:
(Chloroform/Methanol/(1 M Natriumacetat in 25 proz. Ammoniak 70:40:10)

### Beispiel 15

### 3-[[(Hexadecyloxy)hydroxyphosphenyl]oxy]-P,P,P-trimethylpropylphosphonium inneres Salz

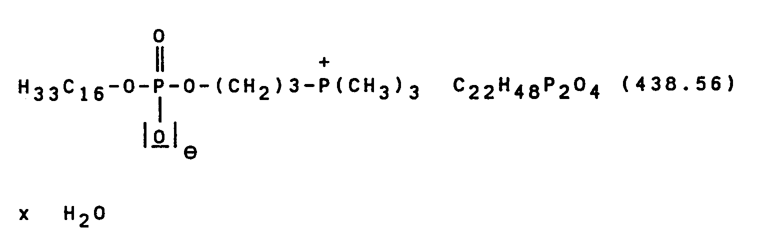

Darstellung analog Beispiel 1 aus 4,2 ml (45 mmol) Phosphoroxychlorid 10,2 g (42 mmol) Hexadecanol 14 + 18 ml Pyridin und 12,0 g (56 mmol) Trimethyl-(3-hydroxypropyl-phosphoniumbromid. Reinigung durch Behandeln mit 55 g Ionenaustauscher Amberlite MBB in 96 proz. Ethanol und anschließender Säulenchromatographie an Kieselgel mit CH₂Cl₂/CH₃OH/25 proz. Ammoniak 70:40:10.
Ausbeute: 1,86 g (10 %)

| Elementaranalyse: | | |
|---|---|---|
| | C | H |
| ber. | 57,87 % | 11,04 % |
| gef. | 57,30 % | 11,20 % |
| | 57,02 % | 11,29 % |

R_{F} = 0,50
Dünnschichtchromatogramm:
(Chloroform/Methanol/1m Natriumacetat in 25 proz. Ammoniak 70:40:10)

### Beispiel 16

### 3-[[(Nonadecyloxy)hydroxyphosphenyl]oxy]-P,P,P-trimethylethylphosphonium inneres Salz

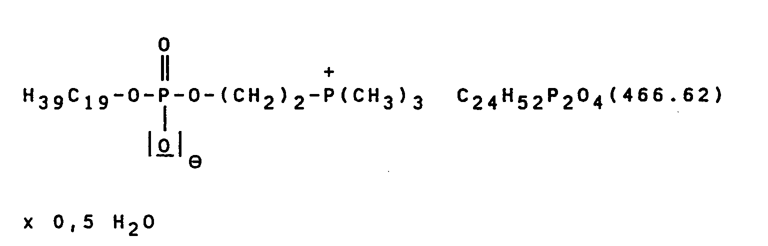

Darstellung analog Beispiel 1 aus 3,2 ml (35 mmol) Phosphoroxychlorid 9,18 g (32 mmol) Monodecanol 11 + 14 ml Pyridin und 8,64 g (43 mmol) Phosphocholinbromid. Reinigung durch Behandeln mit 25 g Ionenaustauscher Amberlite MBB in 96 proz. Ethanol und anschließendem zweimaligem Auskochen mit Aceton.
Ausbeute: 1,85 g (12 %)

| Elementaranalyse: | | |
|---|---|---|
| | C | H |
| ber. | 60,61 % | 11,23 % |
| gef. | 60,69 % | 11,55 % |
| | 61,01 % | 11,39 % |

R_{F} = 0,58
Dünnschichtchromatogramm:
(Chloroform/Methanol/1m Natriumacetat in 25 proz. Ammoniak 70:40:10)

### Beispiel 17

### 2-[[(Eicosyloxy)hydroxyphosphenyl]oxy]-P,P,P-trimethylethylphosphonium inneres Salz

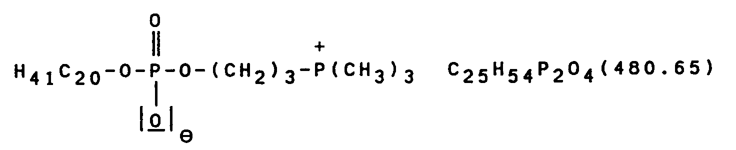

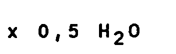

Darstellung analog Beispiel 1 aus 3,2 ml (35 mmol) Phosphoroxychlorid 9,63 g (32 mmol) Eicosanol 11 + 14 ml Pyridin und 8,64 g (43 mmol) Phosphocholinbromid. Reinigung durch Behandeln mit 25 g Ionenaustauscher Amberlite MBB in 96 proz. Ethanol und anschließendem zweimaligem Auskochen in Aceton.
Ausbeute: (0.02 g (7 %)

| Elementaranalyse: | | |
|---|---|---|
| | C | H |
| ber. | 61,32 % | 11,32 % |
| gef. | 61,72 % | 11,65 % |
| | 61,55 % | 11,47 % |

R_{F} = 0,53
Dünnschichtchromatogramm:
(Chloroform/Methanol/1m Natriumacetat in 25 proz. Ammoniak 70:40:10)

### Beispiel 18

### 2-[[(Nonadecyloxy)hydroxyphosphenyl]oxy]-As,As,As-trimethylethylarsonium inneres Salz

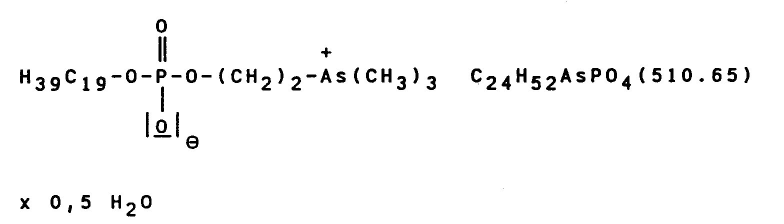

Darstellung analog Beispiel 1 aus 4,6 ml (50 mmol) Phosphoroxychlorid 12,8 g (45 mmol) Nonadecanol, 16 + 20 ml Pyridin und 14,7 g (60 mmol) Arsenocholinbromid. Reinigung durch Behandeln mit 55 g Ionenaustauscher Amberlite MBB in 96 proz. Ethanol.
Ausbeute: 4,05 g (18 %)

| Elementaranalyse: | | |
|---|---|---|
| | C | H |
| ber. | 55,48 % | 10,28 % |
| gef. | 55,89 % | 10,43 % |
| | 55,89 % | 10,52 % |

R_{F} = 0,45
Dünnschichtchromatogramm:
(Chloroform/Methanol/25 proz. Ammoniak 80:25:5)

### Beispiel 19

### 2-[[(Eicozyloxy)hydroxyphosphenyl]oxy]-As,As,As-trimethylethylarsonium inneres Salz

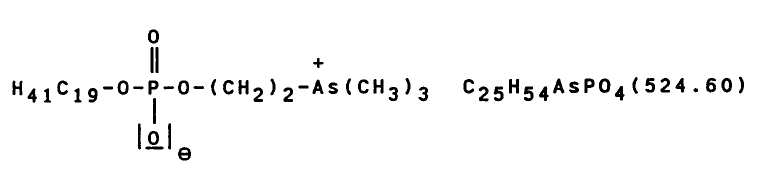

Darstellung analog Beispiel 1 aus 4,6 ml (50 mmol) Phosphoroxychlorid 13,4 g (45 mmol) Eicosanol, 16 + 20 ml Pyridin und 14,7 g (60 mmol) Arsenocholinbromid. Reinigung durch Behandeln mit 50 g Ionenaustauscher Amberlite MBB in 96 proz. Ethanol.
Ausbeute: 2,81 g (12 %)

| Elementaranalyse: | | |
|---|---|---|
| | C | H |
| ber. | 57,24 % | 10,38 % |
| gef. | 56,92 % | 10,52 % |
| | 57,02 % | 10,36 % |

R_{F} = 0,45
Dünnschichtchromatogramm:
(Chloroform/Methanol/25 proz. Ammoniak 80:25:5)

### Beispiel 20

### 2-[[(Heptadecyloxy)hydroxyphosphenyl]oxy]-As,As,As-trimethylethylarsonium inneres Salz

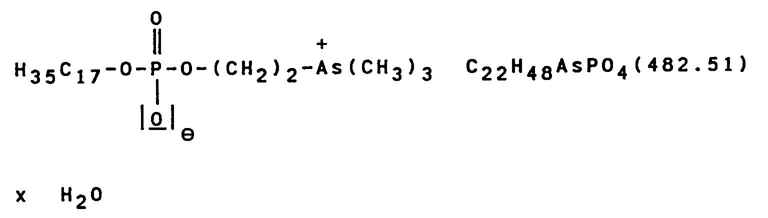

Darstellung analog Beispiel 1 aus 4,6 ml (50 mmol) Phosphoroxychlorid 11,5 g (45 mmol) Heptadecanol, 16 + 20 ml Pyridin und 14,7 g (60 mmol) Arsenocholinbromid. Reinigung durch Behandeln mit 35 g Ionenaustauscher Amberlite MBB in 96 proz. Ethanol und anschließendem Ausrühren in Aceton
Ausbeute: 1,20 g ( 6 %)

| Elementaranalyse: | | |
|---|---|---|
| | C | H |
| ber. | 52,79 % | 10,07 % |
| gef. | 52,46 % | 9,95 % |
| | 53,26 % | 10,30 % |

R_{F} = 0,45
Dünnschichtchromatogramm:
(Chloroform/Methanol/(M Natrtumacetat in 25 proz. Ammoniak 70:40:10).

### Beispiel 21

### 2-[[(Octadecyloxy)hydroxyphosphenylloxy]-P,P-diethyl-P -phenyl-ethylphosphonium inneres Salz

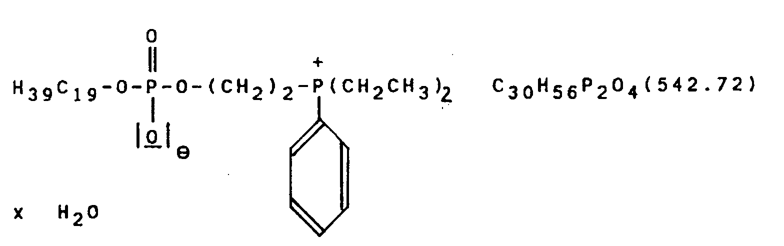

Darstellung analog Beispiel 1 aus 2,3 ml (25 mmol) Phosphoroxychlorid 7,17 g (26,5 mmol) Octadecanol, 8 + 10 ml Pyridin und 7,28 g (26,5 mol) Diethyl-(2-hydroxyethyl)-phenyl-phosphoniumbromid. Reinigung durch Behandeln mit 21 g Ionenaustauscher Amberlite MBB in 96 proz. Ethanol und zweimaliger Umkristallisation aus Aceton.
Ausbeute: 1,76 g (13 %)

| Elementaranalyse: | | |
|---|---|---|
| | C | H |
| ber. | 64,26 % | 10,43 % |
| gef. | 64,03 % | 10,77 % |
| | 64,24 % | 10,92 % |

R_{F} = 0,37
Dünnschichtchromatogramm:
(Chloroform/Methanol/25 proz. Ammoniak 80:25:5)

### Beispiel 22

### 2-[[(cis-13-Docosenyloxy)hydroxyphosphenyl]oxy]-P,P-dtethyl-P-phenyl-ethylphosphontum inneres Salz

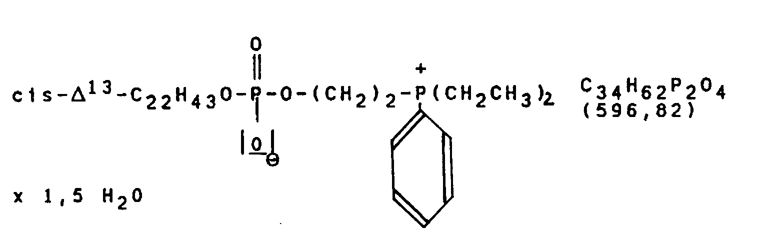

Darstellung analog Beispiel 1 aus 2,3 ml (25 mmol) Phosphoroxychlorid 8,6 g (27 mmol) Erucylalkohol 8 + 10 ml Pyridin und 7,28 g (27 mmol) Diethyl-(2-hydroxyethyl)-phenyl-phosphoniumbromid Behandeln mit 30 g Ionenaustauscher Amberlite MBB in 96 proz. Ethanol und kristallisteren aus Aceton.
Ausbeute: 2,53 g (17 %)

| Elementaranalyse: | | |
|---|---|---|
| | C | H |
| ber. | 65,46 % | 10,50 % |
| gef. | 65,71 % | 10,33 % |
| | 65,59 % | 10,99 % |

R_{F} = 0,37
Dünnschichtchromatogramm:
(Chloroform/Methanol/25 proz. Ammoniak 80:25:5)

### Beispiel 23 (gestrichen)

### Beispiel 24

### 1-0-Octadecyl-2-0-methyl-rac-glycerophosphoarsenocholin

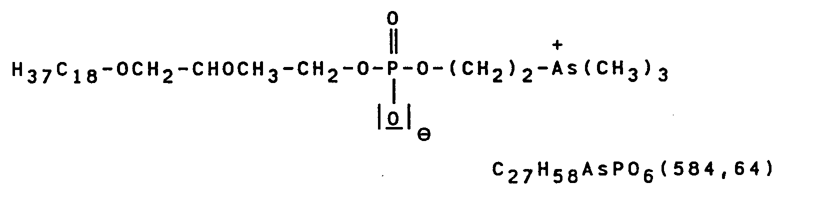

Darstellung analog Beispiel 1 aus 3,0 ml (33 mmol) Phosphoroxychlorid 10,8 g (30 mmol) 1-O-Octadecyl-2-O-methyl-rac-glycerin, 11 + 13 ml Pyridin und 9,8 g (40 mmol) Arsenocholinbromid, Arsenocholinbromid. Reinigung durch Behandeln mit 55 g Ionenaustauscher Amberlite MBB in 96 proz. Ethanol und Ausrühren mit Aceton.
Ausbeute: 5,83 g (33 %)

| Elementaranalyse: | | |
|---|---|---|
| | C | H |
| ber. | 55,47 % | 10,00 % |
| gef. | 55,23 % | 10,23 % |
| | 55,79 % | 10,26 % |

R_{F} = 0,57
Dünnschichtchromatogramm:
(Chloroform/Methanol/1 M Natriumacetat in 25 proz. Ammoniak 70:40:10)

### Beispiel 25

### 1-0-Octadecyl-2-0-methyl-rac-glycerophosphophosphocholin

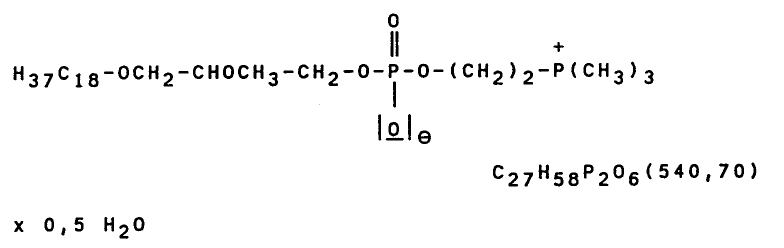

Darstellung analog Beispiel 1 aus 4,6 ml (50 mmol) Phosphoroxychlorid 16,1 g (45 mmol) 1-O-Octadecyl-2-O-methyl-rac-glycerin, 16 + 20 ml Pyridin und 12,1 g (60 mmol) Phosphocholinbromid. Reinigung durch Behandeln mit 45 g Ionenaustauscher Amberlite MBB in 96 proz. Ethanol und Ausrühren mit Aceton.
Ausbeute: 6,25 g (26 %)

| Elementaranalyse: | | |
|---|---|---|
| | C | H |
| ber. | 58,99 % | 10,82 % |
| gef. | 58,73 % | 11,19 % |
| | 59,12 % | 11,05 % |

R_{F} = 0,52
Dünnschichtchromatogramm:
(Chloroform/Methanol/1 M Natriumacetat in 25 proz. Ammoniak 70:40:10)

### Beispiel 26

### 2-[[(Octadecycloxy)hydroxyphosphenyl]oxy]-Sb,Sb,Sb-trimethylethylstibonium inneres Salz

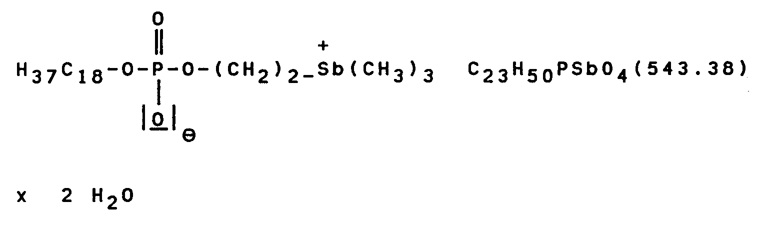

Darstellung analog Beispiel 1 aus 2,1 ml (23 mmol) Phosphoroxychlorid 6,49 g (24,5 mmol) Octadecanol, 7 + 9 ml Pyridin und 7,0 g (24 mmol) Stibonocholinbromid. Reinigung durch Behandeln mit 15 g Ionenaustauscher Amberlite MBB und anschließender Säulenchromatographie an Kielselgel mit CH₂Cl₂/CH₃OH/ 25 proz. Ammoniak 80:25:5. Die produktenthaltenden Fraktionen werden mit Diethylether ausgezogen und eingeengt.
Ausbeute: 1,0 g ( 8 %)

| Elementaranalyse: | | |
|---|---|---|
| | C | H |
| ber. | 47,68 % | 9,39 % |
| gef. | 47,54 % | 9,45 % |
| | 47,58 % | 9,28 % |

R_{F} = 0,62
Dünnschichtchromatogramm:
(Chloroform/Methanol/(M Natriumacetat in 25 proz. Ammoniak 70:40:10)

## Patentansprüche

1. Verbindungen der allgerneinen Formel I 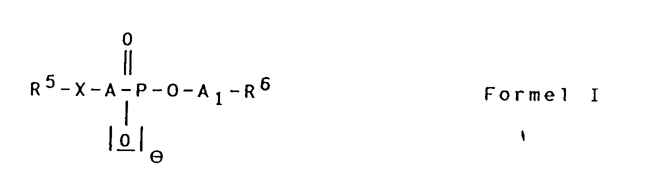
R⁵ = geradkettiger oder verzweigter Alkylrest mit 10-24 Kohlenstoffatomen, der auch eine bis drei Doppel- beziehungsweise Dreifachbindungen enthalten kann,
A = Einfachbindung oder eine der Gruppen mit den Formeln: 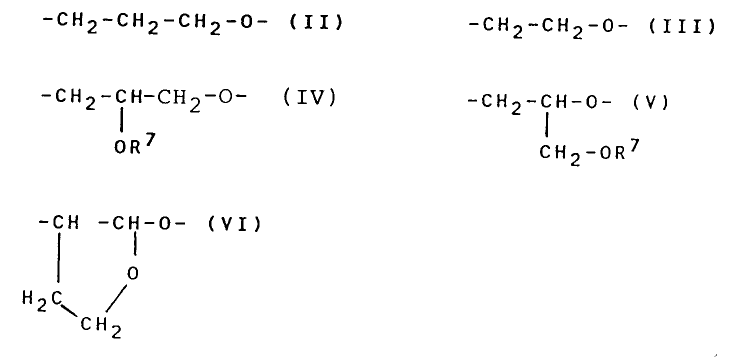
wobei R⁷ = geradkettige Alkylgruppe mit 1 bis 4 Kohlenstoffenatomen; die Gruppen (II) bis (VI) sind so orientiert, daß das Sauerstoffatom an das Phosphoratom der Verbindung (I) gebunden ist.
X = Sauerstoff- oder Schwefelatom oder NH, wenn A = Einfachbindung
X = Sauerstoff- oder Schwefelatom, wenn A eine Verbindung aus den Gruppen der Formeln (II) bis (VI)
A₁ = geradkettiger oder verzweigter Alkylrest mit 2 bis 10 Kohlenstoffatomen,
R⁶ = ⁽⁺⁾YR⁸R⁹R¹⁰ mit R⁸-R¹⁰= geradkettiger, verzweigter oder cyclischer Alkylrest mit 1 bis 6 Kohlenstoffatomen, der gleich oder verschieden sein kann, oder auch Wasserstoff
Y = P, As, Sb oder Bi,
oder eine Gruppe der Formel VII 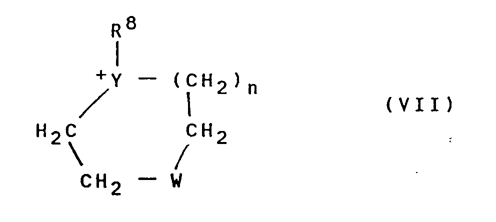
mit n = 0 oder 1
W = CH₂, O, NH oder S; unter der Voraussetzung
n = 1, wenn W nicht gleich CH₂ ist.

2. Verfahren zur Herstellung der Verbindungen gemäß Anspruch 1, Formel I,
dadurch gekennzeichnet,
daß man in an sich bekannter weise Phosphoroxychlorid mit einem n-Alkanol in einem Lösungsmittel oder auch ohne Lösungsmittel umsetzt, das erhaltene Produkt- ohne Isolierung und Reinigung weiter mit einer Verbindung der allgemeinen Formel VIII umsetzt und anschließend hydrolisiert.

3. Verfahren zur Herstellung der Verbindungen gemäß Anspruch 1, Formel I,
dadurch gekennzeichnet,
daß eine Verbindung der allgemeinen Formel IX 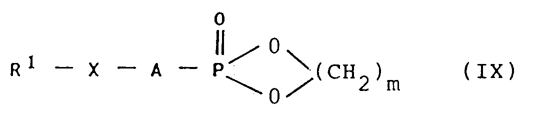 wobei m= 2 oder 3 sein kann, mit einer Verbindung der allgemeinen Formel X oder XI
- YR⁶R⁷R⁸ (X)
oder 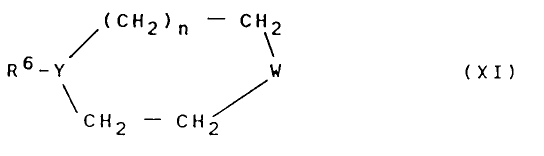 umgesetzt wird.

4. Verfahren zur Herstellung der Verbindungen gemäß Anspruch 1, Formel I,
dadurch gekennzeichnet,
daß eine Verbindung gemäß Formel XIII mit Hydrogenphosphat umgesetzt wird.

5. Verfahren zur Herstellung der Verbindungen gemäß Anspruch 1, Formel I,
dadurch gekennzeichnet,
daß eine Verbindung der Formel XII 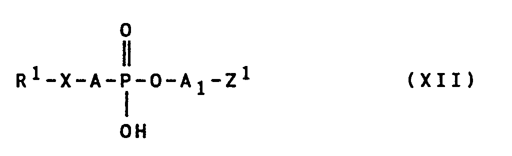
mit Z¹ = Chlor, Brom, Mesylat Tosylat oder Jod
mit Verbindungen der Formel (X) oder (XI) umgesetzt wird.

6. Reinigungsverfahren für die Verbindungen erhältlich gemaß Ansprüchen 2 - 5,
dadurch gekennzeichnet,
daß man das Rohprodukt mit
Mischbettionenaustauscher oder gleichzeitig oder nacheinander mit sauren und/oder basischen Ionenaustauscher behandelt.

7. Verwendung der Verbindungen gemäß Anspruch 1 zur Herstellung von Arzneimitteln zur Behandlung von Tumoren und Protozoenerkrankungen, Haut- und Autoimmunkrankheiten, Viruserkrankungen und bakterielle Infektionen und Bluterkrankungen sowie Osteoporose.

8. Verfahren zur Herstellung von Arzneimitteln zur Behandlung von Tumoren und Protozoenerkrankungen, Haut- und Autoimmunkrankheiten, Viruserkrankungen, bakterielle Infektionen und Bluterkrankungen, dadurch gekennzeichnet, daß man Verbindungen der Formel I in der Menge von 0,1 mg bis 6000 mg mit den üblichen Füll-, Verdünnungs- und Hilfsstoffen zu Arzneimitteln verarbeitet.

9. Arzneimittel zur Behandlung von Tumoren und Protozoenerkrankungen, Haut- und Autoimmunkrankheiten, Viruserkrankungen, bakterielle Infektionen und Bluterkrankungen sowie Osteoporose, dadurch gekennzeichnet, daß es Verbindungen der Formel I in Mengen von 0,1 mg bis 6000 mg enthält.

## Claims

1. Compounds of the general formula I 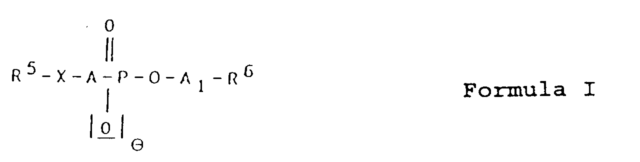
R⁵ = straight-chain or branched alkyl radical having 10-24 carbon atoms, which can also contain one to three double or triple bonds,
A = single bond or one of the groups of the formulae: 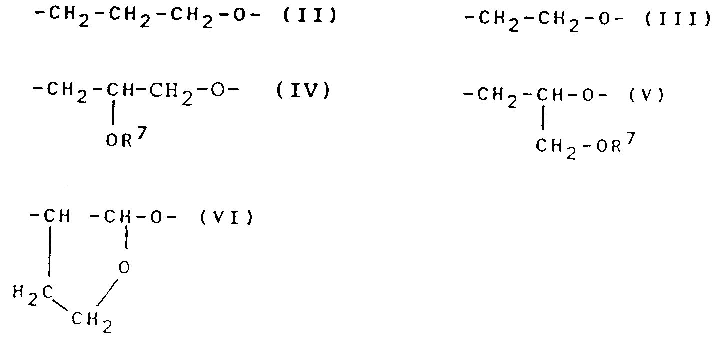 where R⁷ = straight-chain alkyl group having 1 to 4 carbon atoms; the groups (II) to (VI) are oriented such that the oxygen atom is bonded to the phosphorus atom of the compound (I).
X = oxygen or sulphur atom or NH, if A = single bond
X = oxygen or sulphur atom, if A is a compound from the groups consisting of the formulae (II) to (VI)
A₁ = straight-chain or branched alkyl radical having 2 to 10 carbon atoms,
R⁶ = ⁽⁺⁾YR⁸R⁹R¹⁰ where R⁸-R¹⁰ = straight-chain, branched or cyclic alkyl radical having 1 to 6 carbon atoms, which can be identical or different, or alternatively hydrogen
Y = P, As, Sb or Bi,
or a group of the formula VII 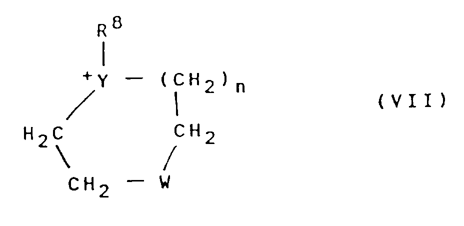
where n = 0 or 1
W = CH₂, O, NH or S; with the condition n = 1 if W is not equal to CH₂.

2. Process for the preparation of the compounds according to Claim 1, formula I, characterized in that, in a manner known per se, phosphorus oxychloride is reacted with an n-alkanol in a solvent or alternatively without solvent, and the product obtained is further reacted - without isolation and purification - with a compound of the general formula VIII and then hydrolysed.

3. Process for the preparation of the compounds according to Claim 1, formula I, characterized in that a compound of the general formula IX 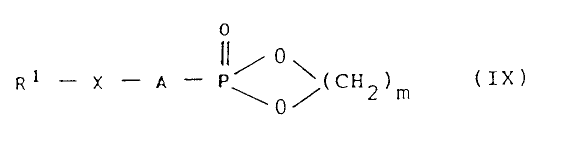 where m can be 2 or 3, is reacted with a compound of the general formula X or XI
- YR⁶R⁷R⁸ (X)
or

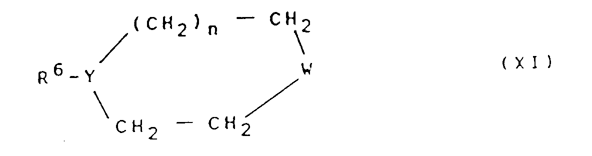

4. Process for the preparation of the compounds according to Claim 1, formula I, characterized in that a compound according to formula XIII is reacted with hydrogen phosphate.

5. Process for the preparation of the compounds according to Claim 1, formula I, characterized in that a compound of the formula XII 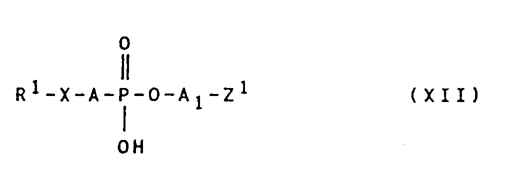 where Z¹ = chlorine, bromine, mesylate, tosylate or iodine is reacted with compounds of the formula (X) or (XI).

6. Purification process for the compounds obtainable according to Claims 2-5, characterized in that the crude product is treated with mixed bed ion exchangers or simultaneously or successively with acidic and/or basic ion exchangers.

7. Use of the compounds according to Claim 1 for the production of medicaments for the treatment of tumours and protozoal disorders, skin and autoimmune diseases, viral disorders and bacterial infections and blood disorders as well as osteoporosis.

8. Process for the production of medicaments for the treatment of tumours and protozoal disorders, skin and autoimmune diseases, viral disorders, bacterial infections and blood disorders, characterized in that compounds of the formula I in an amount from 0.1 mg to 6000 mg are processed with the customary fillers, diluents and auxiliaries to give medicaments.

9. Medicament for the treatment of tumours and protozoal disorders, skin and autoimmune diseases, viral disorders, bacterial infections and blood disorders as well as osteoporosis, characterized in that it contains compounds of the formula I in amounts from 0.1 mg to 6000 mg.

## Revendications

1. Composés de formule générale I 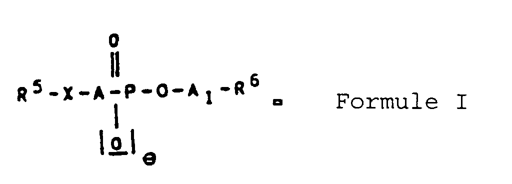
R⁵ = radical alkyle linéaire ou ramifié ayant 10-24 atomes de carbone, qui peut également contenir une à trois doubles, de préférence triples liaisons,
A = liaison simple ou un des groupes de formules: 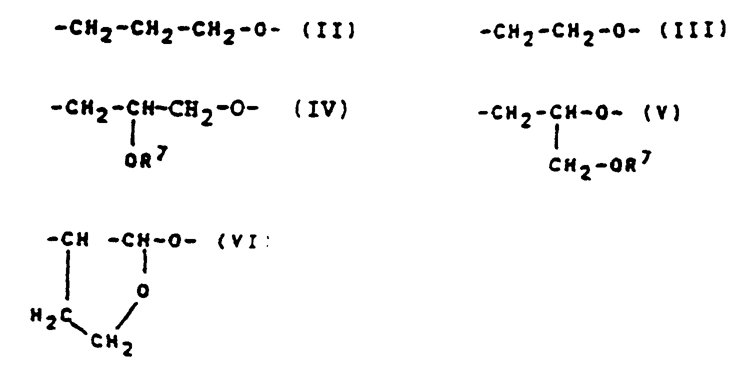
dans lesquelles R⁷ = groupe alkyle linéaire ayant de 1 à 4 atomes de carbone; les groupes (II) à (VI) sont orientés de telle sorte que l'atome d'oxygène soit lié à l'atome de phosphore du composé (I),
X = atome d'oxygène ou de soufre ou NH, si A = liaison simple
X = atome d'oxygène ou de soufre, si A est un composé parmi les groupes de formules (II) à (VI)
A₁ = radical alkyle linéaire ou ramifié ayant de 2 à 10 atomes de carbone,
R⁶ = ⁽⁺⁾YR⁸R⁹R¹⁰ avec R⁸-R¹⁰ = radical alkyle linéaire, ramifié ou cyclique ayant de 1 à 6 atomes de carbone, qui peut être identique ou différent, ou bien un hydrogène
Y = P, As, Sb ou Bi,
ou un groupe de formule VII 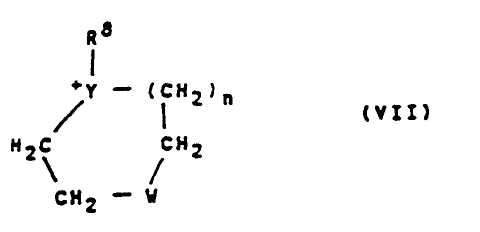
avec n = 0 ou 1
W = CH₂, O, NH ou S; à condition que n = 1, si W est différent de CH₂.

2. Procédé de préparation des composés selon la revendication 1, de formule I, caractérisé en ce que l'on fait réagir, d'une manière connue en soi, de l'oxychlorure de phosphore avec un n-alcanol dans un solvant ou bien sans solvant, l'on fait en outre réagir le produit obtenu - sans isolation et sans purification avec un composé de formule générale VIII et l'on procède ensuite à l'hydrolyse.

3. Procédé de préparation des composés selon la revendication 1, de formule I, caractérisé en ce que l'on fait réagir un composé de formule générale IX 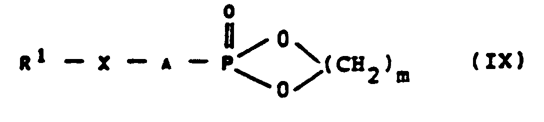 dans laquelle m peut valoir 2 ou 3, avec un composé de formule générale X ou XI
-YR⁶R⁷R⁸ (X)
ou

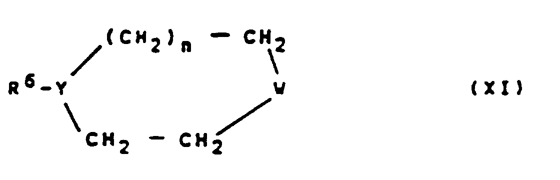

4. Procédé de préparation des composés selon la revendication 1, de formule I, caractérisé en ce que l'on fait réagir un composé de formule XIII avec de l'hydrogénophosphate.

5. Procédé de préparation des composés selon la revendication 1, de formule I, caractérisé en ce que l'on fait réagir un composé de formule XII 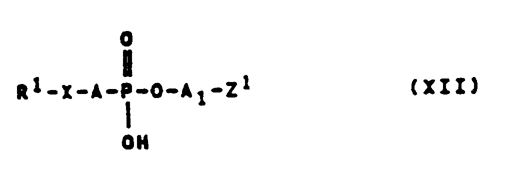 avec Z¹ = chlore, brome, mésylate, tosylate ou iode,
avec des composés de formule (X) ou (XI).

6. Procédé de purification pour les composés pouvant être obtenus selon les revendications 2-5, caractérisé en ce que l'on traite le produit brut par des échangeurs d'ions en lit mélangé, ou simultanément ou successivement par des échangeurs d'ions acides/basiques.

7. Utilisation des composés selon la revendication 1 pour la préparation de médicaments destinés au traitement de tumeurs et d'affections dues à des protozoaires, de maladies de la peau et autoimmunes, d'affections virales et d'infections bactériennes, et d'affections sanguines ainsi que de l'ostéoporose.

8. Procédé de préparation de médicaments destinés au traitement de tumeurs et d'affections dues à des protozoaires, de maladies de la peau et autoimmunes, d'affections virales et d'infections bactériennes, et d'affections sanguines, caractérisé en ce que l'on met en oeuvre des composés de formule I en quantités de 0,1 mg à 6 000 mg avec les charges, diluants et auxiliaires usuels pour former des médicaments.

9. Médicament destiné au traitement de tumeurs et d'affections dues à des protozoaires, de maladies de la peau et auto-immunes, d'affections virales, d'infections bactériennes et d'affections sanguines ainsi que de l'ostéoporose, caractérisé en ce qu'il contient des composés de formule I en quantités de 0,1 mg à 6 000 mg.
